(19) Europäisches Patentamt
European Patent Office
Office européen des brevets

(11) Veröffentlichungsnummer: **0 324 468**
**A2**

(12) **EUROPÄISCHE PATENTANMELDUNG**

(21) Anmeldenummer: **89100473.1**

(51) Int. Cl.4: **C12Q 1/68**

(22) Anmeldetag: **12.01.89**

(30) Priorität: **12.01.88 DE 3800644**

(43) Veröffentlichungstag der Anmeldung:
**19.07.89 Patentblatt 89/29**

(84) Benannte Vertragsstaaten:
**AT BE CH DE ES FR GB GR IT LI LU NL SE**

(71) Anmelder: **BOEHRINGER MANNHEIM GMBH**
**Patentabteilung, Abt. E Sandhofer Strasse**
**112-132 Postfach 31 01 20**
**D-6800 Mannheim 31 Waldhof(DE)**

(72) Erfinder: **Höltke, Hans-Joachim, Dr.rer.nat.**
**Kirchenstrasse 1**
**D-8132 Tutzing(DE)**
Erfinder: **Seibl, Rudolf, Dr.rer.nat.**
**Saalangerstrasse 46**
**D-8122 Penzberg(DE)**
Erfinder: **Kessler, Christoph, Dr.rer.nat.**
**Fraunhofer Strasse 12**
**D-8000 München 5(DE)**
Erfinder: **Mattes, Ralf, Prof.Dr.rer.nat.**
**Friedrich-Zundel-Strasse 14**
**D-7000 Stuttgart 75(DE)**
Erfinder: **Graf, Hermann, Dr.rer.nat.**
**Johannes-Tanner-Strasse 2a**
**D-8000 München 60(DE)**

(74) Vertreter: **Huber, Bernhard, Dipl.-Chem. et al**
**Möhlstrasse 22 Postfach 860 820**
**D-8000 München 86(DE)**

(54) **Verfahren zum hochspezifischen Nachweis von Nukleinsäuren in Lösung.**

(57) Zum Nachweis von Nukleinsäuren definierter Sequenz durch Hybridisierung mit zwei in gleicher Lösungsphase vorliegenden, zu unterschiedlichen Bereichen der nachzuweisenden Nukleinsäure komplementären, einzelsträngigen Nukleinsäuresonden, wobei die eine Nukleinsäuresonde als Detektorsonde dient und über eine chemische Verbindung mindestens ein Hapten als Markierung gebunden enthält, und die andere Nukleinsäuresonde als Einfangsonde dient und an eine feste Matrix gebunden wird, verwendet man als Hapten ein Steroid, das an mindestens eine Position der Detektorsonde, die nicht an der Wasserstoffbrückenbildung mit der nachzuweisenden Nukleinsäure beteiligt ist, über eine Brücke von mindestens vier Atomen Länge kovalent gebunden ist, inkubiert die in Lösung vorliegende, nachzuweisende Nukleinsäure mit der Detektorsonde und der Einfangsonde in beliebiger Reihenfolge, wobei man die Bindung der Einfangsonde an die Matrix vor, während oder nach der Inkubation mit der nachzuweisenden Nukleinsäure bewirkt, trennt die verbleibende Lösung von dem matrixgebundenen Komplex aus Einfangsonde, nachzuweisender Nukleinsäure und Detektorsonde ab und weist den Komplex über einen seinerseits markierten Anti-Hapten-Antikörper nach.

EP 0 324 468 A2

FIG.1

Dig-11(16)-dUTP

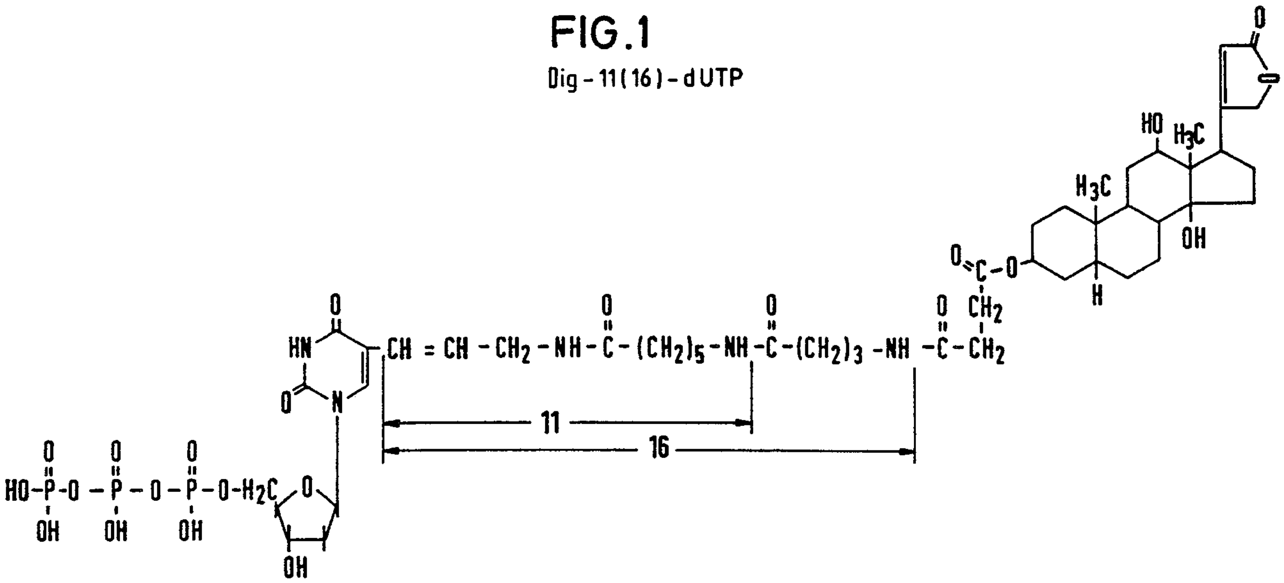

## Verfahren zum hochspezifischen Nachweis von Nukleinsäuren in Lösung

Die Erfindung betrifft ein Verfahren zum Nachweis von Nukleinsäuren definierter Sequenz durch Hybridisierung mit zwei in gleicher Lösungsphase vorliegenden, zu unterschiedlichen Bereichen der nachzuweisenden Nukleinsäure komplementären, einzelsträngigen Nukleinsäuresonden, wobei die eine Nukleinsäuresonde als Detektorsonde dient und über eine chemische Verbindung mindestens ein Hapten als Markierung gebunden enthält, und die andere Nukleinsäuresonde als Einfangsonde dient und an eine feste Matrix gebunden wird.

Eine der meist angewandten molekular-biologischen Techniken ist die DNA/DNA-, RNA/RNA- bzw. RNA/DNA-Hybridisierung zum Nachweis homologer Nukleinsäuresequenzen. Hierzu wird die nachzuweisende Nukleinsäure denaturiert und auf einem Filter fixiert, worauf der Nachweis über die Hybridisierung mit einer markierten komplementären Nukleinsäuresonde durchgeführt wird. Bei der Hybridisierung kommt es zur Ausbildung von Hybrid-Doppelsträngen, bestehend aus Einzelsträngen komplementärer Sequenz der nachzuweisenden Nukleinsäure und der Nukleinsäuresonde. Der Nachweis erfolgt sodann über die Markierung der komplementären Nukleinsäuresonde. Zur Markierung der Sonde wurde bisher meist der Einbau radioaktiv derivatisierter Desoxyribonukleosidtriphosphate angewandt. Der Nachweis der Hybride erfolgte sodann über eine Autoradiographie. Aufgrund der Probleme der Entsorgung radioaktiver Verbindungen sowie der weiteren im Umgang mit radioaktiven Verbindungen einhergehenden Risiken wurde eine nicht radioaktive Art der Markierung von Nukleinsäuresonden entwickelt. Diese Markierung erfolgte beispielsweise über die Biotin/(Strept)-Avidin-Bindung oder eine Hapten/Anti-Hapten-Antikörper-Bindung und daran gekoppelte Markerenzymkonjugate. Der Nachweis des Hybridisierungsproduktes erfolgt in diesem Fall durch die Bestimmung der enzymatischen Aktivität des Markerenzyms über gekoppelte Farbstoffsysteme. Die nicht radioaktiven Systeme, ausgenommen das Biotin/( Strept)-Avidin-System, weisen jedoch eine wesentlich geringere Nachweissensitivität im Vergleich zu radioaktiven Systemen auf, nämlich eine Verminderung der Sensitivität um mindestens den Faktor 10. Beim Biotin/(Strept)-Avidin-System liegt bei direktem Nachweis von Nukleinsäuren mittels Biotin-markierter Nukleinsäure-Sonden die erreichbare Nachweissensivität im gleichen Bereich wie bei radioaktiver Markierung, nämlich im Nachweis von 1 bis 0,1 pg DNA im Dot-blot und im Nachweis von "single copy"-Genen im genomischen Blot in 10 bis 1 $\mu$g genomischer DNA-Fragmente. Die Nachweissensitivität in gekoppelten Nachweissystemen in Lösung liegt jedoch auch bei Verwendung des Biotin/(Strept)-Avidin-Indikatorsystems deutlich niedriger.

Ein großer Nachteil der bisher verwendeten Filterbindung der nachzuweisenden Nukleinsäuresequenzen liegt aber darin, daß bedingt durch die adsorptive Bindung der einzelsträngigen Nukleinsäure an den Filter nur noch eine unvollständige Hybridisierung mit der Nukleinsäuresonde stattfinden kann, da ein erheblicher Teil der vorhandenen Nukleinsäure für die Hybridisierung unzugänglich wird und unter Umständen nur noch 10 bis 30% der Gesamt-Nukleinsäure zum eigentlichen Nachweis zur Verfügung stehen. Zusätzlich dazu wird der genaue Nachweis durch unspezifische Bindung der markierten Nukleinsäuresonde an den Filter erschwert.

Die DE-OS 35 46 312 beschreibt daher ein gekoppeltes Nachweisverfahren, bei dem zum Nachweis von Nukleinsäuren in Lösung mit zwei Nukleinsäuresonden gearbeitet wird, wobei eine der Sonden als Detektorsonde und die andere als Einfangsonde dient.

Der gebildete Komplex aus der nachzuweisenden Nukleinsäure und der Einfangs- und Detektorsonde wird hierbei über eine an die Einfangsonde gekoppelte Komponente, die als Partner in einem Affinitätspaar fungieren kann und über den anderen adsorptiv an die Matrix gebundenen Partner des Affinitätspaares aus dem Hybridisierungsgemisch isoliert und über die Detektorsonde, die einen Indikatorstoff enthält, identifiziert. Die Nachweissensitivität liegt im ng-Bereich. Als Indikatorstoff für die Detektorsonde in einer solchen Sandwich-Hybridisierung werden unter anderem immunologisch nachweisbare Haptene genannt. Haptene und die entsprechenden Anti-Hapten-Antikörper weisen jedoch eine wesentlich geringere Bindungskonstante auf ($K = 2 \times 10^8$ $Mol^{-1}$-$7 \times 10^9$ $Mol^{-1}$; Hunter et al., J. Immunol. Vol. 129, Nr. 3 (1982) 1165) als dies bei Biotin/(Strept)-Avidin ($K = 10^{15}$ $Mol^{-1}$; Green, N.M. (1975) Adv. Protein Chem. 29, 85-133; Chaet, L., Wolf, Fig. (1964) Arch. Biochem. Biophys. 106, 1-5) der Fall ist (ca. Faktor 105 geringer). Die Ausnützung der Biotin/(Strept)-Avidin-Wechselwirkung für die Detektionsreaktion hat jedoch den entscheidenden Nachteil, daß sie sehr störanfällig ist, da das Vitamin Biotin in nahezu allen biologischen Materialien vorkommt.

Der Erfindung liegt daher die Aufgabe zugrunde, eine hochsensitive nicht-radioaktive Nachweismöglichkeit für Nukleinsäuren in Lösung bereitzustellen, die die obengenannten Nachteile vermeidet.

Diese Aufgabe wird erfindungsgemäß gelöst durch ein Verfahren zum Nachweis von Nukleinsäuresequenzen durch Hybridisierung mit zwei in gleicher Lösungsphase vorliegenden, zu unterschiedlichen Bereichen der nachzuweisenden Nukleinsäure komplementären, einzelsträngigen Nukleinsäuresonden, wobei die eine Nukleinsäuresonde als Detektorsonde dient und über eine chemische Verbindung mindestens ein Hapten als Markierung gebunden enthält, und die andere Nukleinsäuresonde als Einfangsonde dient und an eine feste Matrix gebunden wird, welches dadurch gekennzeichnet ist, daß man als Hapten ein Steroid verwendet, das an mindestens eine Position der Detektorsonde, die nicht an der Wasserstoffbrükkenbildung mit der nachzuweisenden Nukleinsäure beteiligt ist, über eine Brücke von mindestens vier Atomen Länge kovalent gebunden ist, die in Lösung vorliegende, nachzuweisende Nukleinsäure mit der Detektorsonde und der Einfangsonde in beliebiger Reihenfolge inkubiert, wobei die Bindung der Einfangsonde an die Matrix vor, während oder nach der Inkubation mit der nachzuweisenden Nukleinsäure bewirkt wird, die verbleibende Lösung von dem matrixgebundenen Komplex aus Einfangsonde, nachzuweisender Nukleinsäure und Detektorsonde abtrennt und den Komplex über einen seinerseits markierten Anti-Hapten-Antikörper nachweist.

Bisher ging man davon aus, daß aufgrund der großen Bindungskonstante der Biotin/(Strept)-Avidin-Wechselwirkung ($K = 10^{15}$ $Mol^{-1}$) alle alternativen, spezifischen Wechselwirkungen mit kleineren Bindungskonstanten weniger sensitiv seien. Außerdem erscheint die Biotin/(Strept)-Avidin-Wechselwirkung durch das Vorhandensein von vier Biotin-Bindungsstellen auf (Strept)-Avidin begünstigt. Daher wurden bisher in nichtradioaktiven Nukleinsäure-Nachweissystemen die Biotin/(Strept)-Avidin-Wechselwirkungen bevorzugt für die Detektionsreaktion verwendet.

Erfindungsgemäß gelingt es jedoch, Nukleinsäuren spezifisch mit mindestens gleicher Sensitivität wie mit Biotin-Streptavidin nachzuweisen, wobei die Selektivität dadurch erhöht wird, daß sowohl für die Matrixbindung als auch für die Detektionsreaktion alternative, spezifische Wechselwirkungsprinzipien eingesetzt werden, die jeweils eine hohe Nukleinsäurenachweis-Sensitivität aufweisen. Die Bindung von nachzuweisender Nukleinsäure in Abwesenheit von entweder Einfangsonde oder Detektorsonde führt zu keinem Nachweis-Signal.

Ein wesentlicher Vorteil des erfindungsgemäßen Verfahrens besteht weiter darin, daß bei der Bestimmung bedeutend weniger unspezifische Bindungen auftreten und daher die Genauigkeit der Bestimmung erhöht und der unspezifische Hintergrund erniedrigt wird.

Die Reihenfolge der Bindung der Detektor- und Einfangsonde an die nachzuweisende Nukleinsäure, sowie der Matrixbindung der Einfangsonde ist beliebig. Es ist also unter anderem möglich, zuerst gleichzeitig die nachzuweisende Nukleinsäure mit der Detektorsonde und der Einfangsonde zu inkubieren und dann den gebildeten Komplex aus den drei Nukleinsäuren an die Matrix zu binden, eine weitere Möglichkeit ist die Bindung der Einfangsonde an die Matrix, gleichzeitig jedoch räumlich getrennt Bindung der nachzuweisenden Nukleinsäure an die Detektorsonde, und anschließendes Inkubieren des Komplexes aus nachzuweisender Nukleinsäure und Detektorsonde mit der matrixgebundenen Einfangsonde. Auch kann zuerst die Einfangsonde an die Matrix gebunden werden und dann im selben Reaktionsgefäß mit der nachzuweisenden Nukleinsäure und der Detektorsonde inkubiert werden. Jegliche weitere Reihenfolge der Inkubation der Nukleinsäuren und der Matrix ist ebenfalls geeignet.

In einer bevorzugten Ausführungsform der Erfindung werden als Steroid Digoxigenin oder Digoxin verwendet. Überraschenderweise zeigen diese beiden Steroide als Hapten gleich hohe Sensitivität im Nachweis von Nukleinsäuren wie das Biotin/(Strept)-Avidin-System, obwohl die K-Werte der Bindung dieser Steroide an die entsprechenden Antikörper um etwa den Faktor $10^5$ niedriger liegen als beim Biotin/(Strept)-Avidin-System, und zusätzlich (Strept)-Avidin vier Bindungsstellen für Biotin aufweist.

Daher sind die erfindungsgemäß erreichten Sensitivitäten in dem gekoppelten Nachweissystem mindestens vergleichbar mit den bei Verwendung des Biotin-(Strept)-Avidin Systems erreichbaren Sensitivitäten und weniger störungsanfällig gegenüber unspezifischer Bindung.

Die Länge der Brücke, über die das Hapten an die Detektorsonde gebunden ist, kann zwischen 4 und 32 Atomen betragen. Die Brücke ist hierbei aus Molekülen aufgebaut, die die Atome C, O, S und/oder N enthalten. Eine größere Kettenlänge ist zwar möglich, aber nicht mehr sinnvoll, da dabei mit einer Verschlechterung der Nachweissensitivität zu rechnen ist. In einer bevorzugten Ausführungsform der Erfindung ist das Hapten über eine Brücke von 11 bis 16 Atomen Länge an die Detektorsonde gebunden. Bevorzugt enthält die Brücke mehr hydrophile als hydrophobe Gruppierungen.

In einer bevorzugten Ausführungsform der Erfindung ist die Brücke linear. In einer weiteren bevorzugten Ausführungsform ist die Brücke eine verzweigte Kette und enthält mindestens an einem Kettenende ein Haptenmolekül. Durch das Vorhandensein mehrerer Haptenmoleküle an einer verzweigtkettigen Brücke kann die Nachweissensitivität zusätzlich verstärkt werden. Ein bevorzugtes, Hapten-markiertes Nukleosidtriphosphat ist in der Fig. 1 mit zwei verschiedenen bevorzugten Brückenkettenlängen dargestellt.

Die Bindung des Haptens über die Brücke an die Detektorsonde ist sowohl über eine endständige oder nicht endständige Phosphatgruppe, als auch einen Riboserest oder eine Base der Nukleinsäure-Sonde möglich. Die Bindung des Haptens über die Brücke muß jedoch so erfolgen, daß die Wasserstoffbrückenbildung zwischen den beiden komplementären Nukleinsäuresträngen nicht beeinträchtigt wird. Bevorzugt ist das Hapten über die Brücke an eine Base oder einen Riboserest der Detektorsonde gebunden.

Besonders bevorzugt ist das Hapten über die Brücke an die $C_5$-Position von Uracil oder Cytosin, die $C_8$-Position von Adenin oder Guanin, oder an die 2′-Position der Ribose der Detektorsonde gebunden.

Die Verbindung von Steroidhapten und Brücke ist vorzugsweise eine Ester-, Amid- oder Etherbindung.

Die Einfangsonde kann in beliebiger Weise an die feste Matrix gekoppelt werden. Es sind also koordinative, adsorptive und kovalente Bindung möglich. Die Bindung kann jedoch auch über ein Bindungssystem erfolgen.

In einer bevorzugten Ausführungsform ist die Einfangsonde über ein Bindungssystem koordinativ oder kovalent an die feste Matrix gebunden. Ein solches Bindungssystem besteht aus zwei Komponenten, wobei eine Komponente adsorptiv, koordinativ oder kovalent an die feste Matrix gebunden ist und die zweite Komponente an die Einfangsonde gebunden ist. Durch die Verbindung der beiden Komponenten des Bindungssystems wird gleichzeitig die Einfangsonde an die feste Matrix gekoppelt. Geeignete Bindungssysteme sind z. B. das Biotin-/(Strept)-Avidin-System, Hapten-Antihapten-Antikörpersysteme sowie komplementäre Nukleinsäuresequenzen.

In einer bevorzugten Ausführungsform ist eine Komponente des Bindungssystems über eine Brücke von mindestens vier Atomen Länge an mindestens eine Position der Einfangsonde, die nicht an der Wasserstoffbrückenbildung mit der nachzuweisenden Nukleinsäure beteiligt ist, gebunden.

In einer weiteren bevorzugten Ausführungsform sind die beiden Komponenten des Bindungssystems ein Hapten und ein Anti-Hapten-Antikörper, mit der Maßgabe, daß das Hapten ein von der Markierung der Detektorsonde verschiedenes Hapten ist und der Anti-Hapten-Antikörper nicht mit dem Markierungshapten der Detektorsonde kreuzreagiert.

In einer weiteren bevorzugten Ausführungsform sind die beiden Komponenten des Bindungssystems Biotin und (Strept-)Avidin oder Oligo- oder Poly d(C) und Oligo-oder Poly d(G). Werden als Bindungssystem Oligo- oder Poly d(C)/ Oligo- oder Poly d(G) verwendet, ist es weiter bevorzugt, wenn sich eine der beiden Komponenten direkt am 5′- oder 3′-Ende der Einfangsonde befindet.

Als feste Matrix kann jedes beliebige Material verwendet werden, an das sich adsorptiv, koordinativ oder kovalent die Einfangsonde oder eine Komponente des Bindungssystems fixieren lassen. Bevorzugt verwendet man als feste Matrix einen Nitrocellulosefilter, Nylonfilter, Plastik, Plexiglas oder Nitrocellulose- oder Nylon-beschichtetes Plastik oder Plexiglas. In einer besonders bevorzugten Ausführungsform verwendet man als feste Matrix die Wandung eines Reaktionsgefäßes, welches beispielsweise ein Polyethylenreagenzglas, eine Mikrotiterplatte oder eine Küvette sein kann, wobei sich ein weiterer Vorteil der Erfindung darin zeigt, daß der Nachweis über eine Farbreaktion direkt im Reaktionsgefäß durch Messung der Extinktion bzw. Absorption bei entsprechenden Wellenlängen geführt werden kann.

Die Hybridisierung der Detektor- und Einfangsonde mit der nachzuweisenden Nukleinsäure erfolgt in Lösung bei einer Temperatur, die abhängig von der Länge der Nukleinsäure-Sonden und der daraus resultierenden Schmelztemperatur der zu erwartenden Hybride gewählt wird.

Der Nachweis des nach der Hybridisierung und Matrixbindung gebildeten Komplexes erfolgt über den Nachweis von Hapten-markierter, komplex-gebundener Detektor-Sonde über die Bindung von ihrerseits markierten Anti-Hapten-Antikörpern oder Anti-Hapten-Antikörperfragmenten, mit denen der Komplex inkubiert wird.

Die Markierung des gegen das Hapten der Detektorsonde gerichteten Anti-Hapten-Antikörpers oder eines Fragmentes geschieht in an sich bekannter Weise, bevorzugt sind Enzymmarkierung, radioaktive Markierung, Fluoreszenzmarkierung oder (Bio)-Lumineszenzmarkierung. Besonders bevorzugt wird eine Enzymmarkierung verwendet, wobei wiederum bevorzugt als Markierungsenzym Alkalische Phosphatase, Peroxidase oder $\beta$-Galactosidase verwendet wird. Bevorzugt wird die Bestimmung der katalytischen Aktivität der Markierungssysteme über ein Redoxsystem durchgeführt. Besonders bevorzugt sind hierzu Leukosysteme. Am meisten bevorzugt erfolgt die Bestimmung über indigoide Systeme als oxidierbare Verbindungen und Tetrazoliumsalze als Oxidationsmittel.

In einer bevorzugten Ausführungsform verwendet man als Markierungsenzym Alkalische Phosphatase und führt die Bestimmung über das Redoxsystem X-Phosphat/Nitroblau-Tetrazolium durch. X-Phosphat ist hierbei ein Trivialname für 5-Brom-4-Chlor-3-Indolylphosphat, und Nitroblau-Tetrazolium steht für die Verbindung 3,3′-(3,3′-Dimethoxy-4,4′-Biphenylen)-bis-[5-Phenyl-2-(4-Nitrophenyl)-Tetrazoliumchlorid]. Alkalische Phosphatase spaltet das chromogene Substrat, in diesem Fall X-Phosphat, das durch die Abspaltung des Phosphats und Oxidation ein blaues, schwerlösliches Dimer bildet, wobei gleichzeitig die Tetrazolium-Verbindung zu einem ebenfalls blauen, schwerlöslichen Formazan reduziert wird. Diese Redoxreaktion ist in Fig. 2 dargestellt.

Der Nachweis der anderen geeigneten Bestimmungssysteme wird nach an sich bekannten Methoden durchgeführt.

Zur Synthese von einzelsträngigen Nukleinsäuresonden, welche über eine Brücke mit einem Hapten markiert sind, können verschiedene Methoden angewendet werden.

1. Bei der Phagen-RNA-Polymerase-katalysierten "Transkriptions" Methode (J. Mol. Biol. 166 (1983) 477) wird während der Desoxyribonukleinsäure-abhängigen Ribonukleinsäure-Synthese die gebildete Ribonukleinsäure derivatisiert. Als Phagen-codierte RNA-Polymerase werden z.B. Phage SP6-, T7- oder T3-codierte Enzyme verwendet. Für diese Methode werden doppelsträngige Desoxyribonukleinsäuren verwendet, die SP6-, T7- oder T3-Promotoren enthalten. Durch Zugabe von SP6-, T7- oder T3-RNA Polymerase und aller vier Arten der Ribonukleosid-Triphosphate wird, ausgehend vom homologen Promotor, der zum codogenen Desoxyribonukleinsäure-Strang komplementäre Ribonukleinsäure-Strang, das Transcript, gebildet. Dabei werden die als Substrat angebotenen vier Ribonukleosidtriphosphat-Arten eingebaut. Bis zu vier dieser Triphosphat-Arten sind teilweise oder gänzlich durch Kopplung eines Steroids über eine Brücke derivatisiert, so daß bei der Ribonukleinsäure-Synthese dieses Steroid mit eingebaut wird. Im Fall der Digoxigenin/Digoxin-Markierung werden Digoxigenin/Digoxin-markierte Ribonukleotide, im Fall der Biotin-Markierung werden Biotin-markierte Ribonukleotide verwendet.

2. Für die "Reverse Transkriptions" Methode ist das Ausgangsmaterial einzelsträngige RNA. Retrotranskription mit Reverser Transkriptase führt zum Einbau von Hapten-markierten Nukleotiden in den komplementären DNA-Strang. Als Reverse Transkriptasen werden z.B. Virus AMV- oder Mo-MLV-codierte Enzyme verwendet. Es resultiert ein im DNA-Strang hemi-markiertes DNA/RNA-Hybrid. Der nicht markierte RNA-Strang wird selektiv durch Alkalische Hydrolyse oder Behandlung mit RNase H verdaut.

Im Fall der Digoxigenin/Digoxin-Markierung werden Digoxigenin/Digoxin-markierte Desoxyribonukleotide, im Fall der Biotin-Markierung werden Biotin-markierte Desoxyribonukleotide verwendet.

3. Bei der "Exonuclease III" Methode ist das Ausgangsmaterial doppelsträngige DNA, von der einer der beiden Stränge über Primer-abhängigen Einbau Hapten-derivatisierten Nukleotids mittels DNA-Polymerase (z.B. Klenow Enzym, Phagen-kodierte T4 oder T7 DNA-Polymerase, DNA-Polymerase aus Thermus aquaticus) markiert wurde. Die resultierende hemi-markierte doppelsträngige DNA wird mit zwei unterschiedlichen Class II Restriktionsendonukleasen gespalten, von denen eine Fragmentenden mit 5′-, und die andere Fragmentenden mit 3′-überhängenden Einzelstrangenden erzeugt. Die Auswahl der Endonukleasen ist derart, daß das 5′-überhängende Einzelstrangende dem Hapten-markierten Strang zugeordnet ist, während das 3′-überhängende Einzelstrangende dem unmarkierten Strang zugeordnet ist. Durch anschließenden Abbau mit Exonuklease III wird mit hoher Präferenz nur der nicht markierte DNA-Strang abgebaut, dessen 3′-Ende recessiv, also nicht überstehend ist. 3′-überhängende Fragmentenden werden von Exonuklease III wesentlich langsamer hydrolysiert. Der verbleibende komplementäre Einzelstrang ist Hapten-markiert.

4. Bei der "Lambda-Exonuclease" Methode ist das Ausgangsmaterial eine doppelsträngige DNA, von der einer der beiden Stränge über Primer-abhängigen Einbau Hapten-derivatisierter Nukleotide mittels DNA-Polymerase (z.B. Klenow Enzym, Phagen-kodierte T4 oder T7 DNA Polymerase, DNA-Polymerase aus Thermus aquaticus) markiert wird. Als Primer werden 5′,3′-dephosphorylierte Oligonukleotide, oder anderweitig am 5′-Ende gegen Verdau mit Lambda-Exonuklease geschützte Primer verwendet. Alternativ können geschützte Primer durch Rückfaltung 3′-endständiger, invers repetitiver Sequenzen in der Einzelstrang-DNA erhalten werden, die durch mindestens 5 Basen voneinander getrennt sind (terminale "loops" mit internen 3′-OH-Enden).

Da die resultierende hemi-markierte doppelsträngige DNA nur im unmarkierten Strang phosphorylierte 5′-Fragmentenden hat, wird mit hoher Präferenz nur der markierte Strang durch anschließende Einwirkung mit Lambda-Exonuclease abgebaut. Lambda-Exonuklease hat hohe Präferenz für phosphorylierte 5′-Fragmentenden. Hydroxylierte 5-Fragmentenden werden wesentlich langsamer hydrolysiert.

Der verbleibende komplementäre Einzelstrang ist Hapten-markiert.

5. Bei der "Nick-Translations" Methode sind die Ausgangsmaterialien doppelsträngige DNA-Fragmente. Nach der Bildung eines Nicks spezifisch in einem Strang durch z.B. Phage f1 Protein A und anschließenden Austausch Hapten-markierter Nukleotide durch E. coli DNA Polymerase I wird der Nick-haltige Strang markiert. Die resultierende hemi-markierte doppelsträngige DNA wird wie unter 1. mit 2 unterschiedlichen Class II Restriktionsendonukleasen und Exonuklease III weiterbehandelt.

Angaben für die Methoden 1 bis 5 finden sich in Maniatis, T. et al., Molecular Cloning (1982) Cold Spring Harbor Laboratory, Cold Spring Harbor, New York; Kessler, C., Enzymes in Genetic Engeneering, Ullmann's Encyclopedia of Industrial Research, Vol. A9, VCH Verlagsgesellschaft Weinheim, 1987, Seiten 341 bis 530.

6. In der "photochemischen" Methode (Nucl. Acids Res. 13 (1985) 745-761) wird die Nukleinsäuresonde in Gegenwart von Photo-Digoxigenin (Fig. 3) mit sichtbarem Licht mit UV-Anteil bestrahlt. Unter

Abspaltung von Stickstoff ($N_2$) entsteht ein Nitrenradikal, das kovalent an die Nukleinsäure bindet.

7. Für die "chemische" Methode werden im Rahmen der Oligonukleotidsynthese nach der Phosphit-Triester-Methode neben den geschützten Nukleosid-phosphoramiditen (dA/dG/dC/dT) geschützte, mit substituierbaren Aminofunktionen modifizierte Nukleosid-phosphoramidite (dA/dG/dC/dU) zum gezielten Einbau in den Oligo-Desoxyribo-nukleotid-Einzelstrang verwendet. Die Modifizierung von dC/dU geschieht bevorzugt in Position 5 des Pyrimidin-Ringes, die von dA/dG bevorzugt an Position 8 des Purinmoleküls.

Nach Abschluß der Synthesezyklen und Entfernung der Schutzgruppen resultieren einzelsträngige, an den Nukleobasen mit substituierbaren Aminofunktionen modifizierte Oligo-Desoxyribo-nukleotide, die mit geeigneten Haptenen markierbar sind. Solche Haptene sind Steroide, bevorzugt Digoxigenin, Digoxin, d.h. die Markierung geschieht durch Umsetzung des Oligo-Desoxyribo-nukleotides mit den entsprechenden aktivierten Estern, Amiden oder Ethern der Haptene, vorzugsweise ihren N-hydroxy-succinimidestern.

In einer bevorzugten Ausführungsform der Erfindung werden Hapten-markierte Nukleinsäure-Sonden verwendet, deren Hapten in die Nukleinsäure-Sonde enzymatisch mit Hilfe von RNA-Polymerase, DNA-Polymerase, Exonukleasen oder Reversen Transkriptasen und entsprechenden Haptenmodifizierten Desoxy- oder Ribonukleosidtriphosphat-Substraten eingebaut wurde.

In einer weiteren bevorzugten Ausführungsform der Erfindung werden Hapten-markierte Nukleinsäuresonden verwendet, deren Hapten in die Nukleinsäure-Sonde photochemisch mit Hilfe von Photo-Hapten eingebaut wurde und in einer dritten bevorzugten Ausführungsform werden Nukleinsäuresonden verwendet, deren Hapten in die Nukleinsäure-Sonde chemisch im Rahmen der Oligo-DeSoxyribonukleotid-Synthese durch Einbau geschützter, mit substituierbaren Aminofunktionen modifizierter Nukleosidphosphoamidite und - nach Entfernung der Schutzgruppen - durch Umsetzung des modifizierten Oligo-Desoxyribonukleotids mit aktivierten Estern, Amiden oder Ethern der Haptene eingebaut wurde.

Die folgenden Beispiele in Verbindung mit den Figuren erläutern die Erfindung weiter:

Fig. 1 zeigt ein über eine Brücke von entweder 11 oder 16 Atomen Länge mit Digoxigenin markiertes Deoxyuridintriphosphat;

Fig. 2 zeigt die farbbildende Redoxreaktion des Systems X-Phosphat/Nitroblautetrazolium bei Einwirkung von Alkalischer Phosphatase.

Fig. 3 zeigt schematisch die Darstellung von Photodigoxigenin.

Fig. 4 zeigt die Herstellung eines RNA-Transkripts nach der SP6 bzw. T7-RNA-Polymerase katalysierten Transkriptionsmethode mit dem Plasmid pSPT 18.

Fig. 5 zeigt das Plasmid pSPT 18;

Fig. 6 zeigt die Auswertung der Farbbildungsreaktion eines erfindungsgemäßen Nukleinsäurenachweises.

Fig. 7 zeigt die DNA-Sequenz (A) und die Restriktionskarte (B) von pSPT18.

**Beispiel 1**

Digoxigenin-O-succinyl-[5-(amidoallyl)-2′-desoxy-uridin-5′-Triphosphate]-Tetralithiumsalz

(Dig-4-dUTP)

$C_{39}H_{52}O_{21}N_3P_3Li_4$ MG 1019,5

200 mg Digoxigenin-O-succinyl-N-hydroxysuccinimidester (0,34 mMol) werden in 7 ml Dimethylformamid gelöst und einer Lösung von 186 mg 5-Allylamino-2′-desoxy-uridin-5′-triphosphat-Tetralithiumsalz (0,34 mMol) in 6 ml $H_2O$ zugefügt. Man gibt zu dem Reaktionsgemisch 62 ml 0,1 m Na-boratpuffer, pH 8,5 und rührt ca. 15 Stunden bei Raumtemperatur über Nacht.

Nach dieser Zeit beobachtet man papierelektrophoretisch (0,05 m-Citratpuffer, pH 5,0) unter UV-Licht neben unumgesetztem 5-Allylamino-dUTP einen etwas tiefer laufenderen Fleck des gewünschten Produktes.

Die Aufreinigung geschieht wie unter Beispiel 9 beschrieben.

Ausbeute: 130 mg = 37% der Th.

UV-Spektrum (Phosphatpuffer pH 7,0): Maxima: 220 nm, 290 nm

**Beispiel 2**

Digoxigenin-O-succinyl-ε-amidocapronsäure

$C_{33}H_{49}O_9N$ MG: 603,8

In einem 250 ml-Rundkolben werden 5 g Digoxigenin-O-succinyl-N-hydroxysuccinimidester (8,5 mMol) in 150 ml Dimethylformamid (DMF) gelöst und dazu eine Suspension von 1,12 g 6-Aminocapronsäure (8,5 mMol) und 1,2 ml Triethylamin in 20 ml DMF gegeben. Man rührt über Nacht bei Raumtemperatur magnetisch, wobei allmählich eine homogene Lösung entsteht. Nach dieser Zeit ist die Umsetzung laut Dünnschichtchromatographie (Kieselgel; Essigsäureethylester/Petrolether/Ethanol 1:1:1, Detektion: Besprühen mit einer Mischung von 10 ml Eisessig + 0,2 ml konz. $H_2SO_4$ + 0,1 ml Anisaldehyd und Erhitzen auf 120 °C bis zum Erscheinen blauschwarzer Flecke; $R_f$ ca. 0,7; $R_f$ Digoxigenin-OSu-ester ca. 0,85) praktisch vollständig.

Man destilliert DMF im Hochvakuum restlos ab und löst das verbleibende Öl in 50 ml $H_2O$ unter Zugabe von konz. Ammoniaklösung. Dann wird durch Zufügen von 225 ml wäßriger Zitronensäurelösung (100 g Zitronensäure/1) die "freie" Digoxigeninamidocapronsäure abgeschieden. Die harzig-zähe Masse wird durch Anreiben mit Wasser fest; man saugt ab, wäscht mehrfach mit $H_2O$ nach und trocknet letztlich über $P_2O_5$ im Ölpumpenvakuum.

Ausbeute: 3,45 g = 68% der Th.

**Beispiel 3**

Digoxigenin-O-succinyl-ε-amidocapronsäure-N-hydroxysuccinimidester

$C_{37}H_{52}O_{11}N_2$ MG: 700,8

In einem 100 ml-Rundkolben werden 3,45 g Digoxigenin-O-succinyl-ε-amidocapronsäure (5,7 mMol) in 20 ml wasserfreiem Dimethylformamid (DMF) gelöst, und nacheinander mit 0,7 g N-Hydroxysuccinimid (6 mMol), sowie 1,3 g Dicyclohexylcarbodiimid (6,3 mMol) versetzt. Man rührt über Nacht bei Raumtemperatur, saugt anderntags vom abgeschiedenen Dicyclohexylharnstoff ab und zieht das DMF im Ölpumpenvakuum ab. Das zurückbleibende Öl wird in 20 ml Essigsäureethylester aufgenommen und in ca. 150 ml eiskalten (-20 °C) Petrolether eingerührt. Das ausgefallene, anfangs noch harzig-zähe Produkt reibt man mehrfach mit eiskaltem trockenem Petrolether bis zum Festwerden durch. Nach Trocknung über $P_2O_5$ im Vakuum erhält man

3,35 g = 84 % der Th.

Elementaranalyse:

| | | |
|---|---|---|
| C ber.: 63,4% | H ber.: 7,5% | N ber.: 4,0 % |
| C gef.: 63,1% | H gef.: 7,7% | N gef.: 4,07%. |

Beispiel 4

Digoxigenin-O-succinyl-ε-amidocaproyl-[5-(amidoallyl) -2′-desoxy-uridin-5′-triphosphat]-Tetranatriumsalz

(Dig-11-dUTP)

$C_{45}H_{63}O_{22}N_4P_3Na_4$ MG: 1196,7

260 mg Digoxigenin-O-succinyl-ε-amidocapronsäure-N-hydroxysuccinimidester (0,37 mMol) werden in 7 ml DMF gelöst und zu einer Lösung von 200 mg 5-Allylamino-2′-desoxy-uridin-5′-triphosphat-Tetralithiumsalz (0,37 mMol) in 6 ml $H_2O$ gegeben. Man fügt dem Gemisch 62 ml 0,1 m-Natriumboratpuffer, pH 8,5 zu und rührt bei Raumtemperatur über Nacht (ca. 15 Stunden).

In der Papierelektrophorese (0,05 m-Citratpuffer, pH 5,0) beobachtet man im UV-Licht nach dieser Zeit neben etwas unumgesetztem Allylamino-dUTP einen etwas tiefer laufenderen Fleck der gewünschten Verbindung (alternativ: Dünnschichtchromatographie (DC) auf Kieselgel, Fließmittel Isobuttersäure/konz. Ammoniaklösung/$H_2O$ = 66:1:33, Detektion im UV oder Besprühen mit Anisaldehyd-Reagenz - siehe Beispiel 2 -; $R_f$-Werte: 5-Allylamino-dUTP 0,2; Dig-amidocapronsäure-OSu-ester 0,7; Dig-11-dUTP 0,45).

Zur Aufreinigung wird das Reaktionsgemisch im Ölpumpenvakuum bis zum festen Rückstand eingedampft, in 200 ml $H_2O$ aufgenommen und auf eine Ionenaustauschersäule (DEAE-Sephadex A25, $HCO_3^-$-Form, Säulendimension 1,5 x 30 cm) gegeben. Nach Aufziehen wird kurz mit Wasser gewaschen, dann mit einem Gradient von je 1 1 $H_2O$ auf 0,4 m TEAB (Triethylammoniumbicarbonat), pH 8 eluiert. Die reines Produkt enthaltenden Fraktionen werden vereinigt, im Vakuum konzentriert und durch mehrfaches Eindampfen mit Methanol von überschüssigem TEAB befreit (kein Geruch von freiem Triethylamin mehr!). Man nimmt den Kolbeninhalt in wenigen ml Wasser auf, passiert die Lösung über eine kurze Kationenaustauschersäule DOWEX 50 WS8 (1 x 10 cm) in der $Na^+$-Form, wäscht die Säule bis zur ODE-Freiheit des Waschwassers (Messung im UV bei 240 nm) und dampft im Vakuum bis auf ca. 20 ml ein. Nach Lyophilisation werden 200 mg (45% der Th.) Dig-11-dUTP-$Na_4$ als weißes Pulver erhalten. <u>Analytik:</u> $H_2O$-Bestimmung: 7,9%

<u>Elementaranalyse:</u> (unter Berücksichtigung des $H_2O$-Gehaltes):

| | | | |
|---|---|---|---|
| C ber.: 41,8% | H ber.: 5,3% | N ber.: 4,3% | P ber.: 7,2% |
| C gef.: 41,08% | H gef.: 5,35% | N gef.: 4,7% | P gef.: 7,1%. |

<u>UV-Spektrum</u> (Phosphatpuffer pH 7,0): Maxima: 220 nm, 290 nm.

**Beispiel 5**

Digoxigenin-O-succinyl-γ-amidobuttersäure

$C_{31}H_{45}O_9N$ MG: 575,8

Die Verbindung wird durch Umsetzung von 3 g Digoxigenin-O-succinyl-N-hydroxysuccinimidester (5,1 mMol) mit 0,63 g 4-Aminobuttersäure (6,1 mMol) wie in Beispiel 1 für das Capronsäurederivat beschrieben, hergestellt.

Nach erfolgter Umsetzung wird das Reaktionsgemisch im Vakuum eingedampft, der Rückstand in $H_2O$-Methanol (20%) gelöst und über eine Kationenaustauschersäule (DOWEX 50 WX8) in der $H^+$-Form gegeben. Eluat und Waschwasser (pH ca. 4) werden eingedampft, der zurückbleibende schmierig-zähe Rückstand in n-Butanol gelöst und dreimal mit Wasser ausgeschüttelt. Die Butanol-Phase enthält das gewünschte Produkt und wird nach Abziehen des Butanols und dreimaliger Codestillation mit wasserfreiem DMF (Entfernung restlichen Wassers) direkt zur Weiterverarbeitung zum entsprechenden N-Hydroxysuccinimidester (Beispiel 6) eingesetzt. <u>Ausbeute:</u> 2,94 g (Öl)

**Beispiel 6**

Digoxigenin-O-succinyl-γ-amidobuttersäure-N-hydroxy-succinimidester

$C_{35}H_{48}O_{11}N_2$ MG: 672,8

2,94 g Digoxigenin-O-succinyl-γ-amidobuttersäure ca. 5,1 mMol) werden als Öl aus Beispiel 5 mit 0,62 g N-Hydroxysuccinimid (5,4 mMol) und 1,16 g Dicyclohexylcarbodiimid (5,6 mMol) in 20 ml wasserfreiem DMF wie unter Beispiel 3 beschrieben, umgesetzt und aufgearbeitet. Der resultierende Hydroxysuccinimidester wird als Öl - wie in Beispiel 7 beschrieben - mit ε-Aminocapronsäure umgesetzt. Ausbeute: 3,46 g (Öl)

**Beispiel 7**

Digoxigenin-O-succinyl-γ-amidobutyryl-ε-amidocapronsäure

$C_{37}H_{56}O_{10}N_2$ MG: 689,0

In einem 250 ml-Rundkolben werden 0,8 g -Aminocapronsäure (6,2 mMol) und 0,75 ml Triethylamin in 12 ml DMF suspendiert und dazu eine Lösung von 3,46 g Digoxigenin-O-succinyl-γ-amidobuttersäure-N-hydroxysuccinimidester (5,1 mMol, Öl aus Beispiel 6) in 90 ml DMF gegeben. Man läßt über Nacht ca. 15 Stunden bei Raumtemperatur rühren, wonach eine nahezu homogene Lösung entstanden ist. Laut DC (Bedingungen siehe unter Beispiel 2) ist die Umsetzung fast quantitativ.

Die Aufarbeitung geschieht wie unter Beispiel 5 beschrieben (Überführung in die "freie" Carbonsäure durch DOWEX 50-Chromatographie, Extraktion mit n-Butanol). Die Butanol-Phase enthält neben dem gesuchten Produkt noch etwas polareres und unpolareres Material und wird deswegen durch Chromatographie an Kieselgel 60 (Säule 40x3cm, Elutionsmittel Essigsäurethylester/Petrolether 50/75/Ethanol 1:1:1) gereinigt. Nach Vereinigen der sauren Fraktionen und Eindampfen erhält man als Öl 1,48 g = 42% der Th.

**Beispiel 8**

Digoxigenin-O-succinyl-γ-amidobutyryl-ε-amidocapronsäure-N-hydroxysuccinimid-ester

$C_{41}H_{59}O_{12}N_3$ MG: 785,8

0,2 g Digoxigenin-O-succinyl-γ-amidobutyryl-ε-amidocapronsäure (Öl aus Beispiel 7, ca. 0,29 mMol) werden mit 0,034 g N-hydroxysuccinimid (0,3 mMol) und 66 mg Dicyclohexylcarbodiimid (0,32 mMol) in 8 ml wasserfreiem DMF wie unter Beispiel 3 beschrieben, umgesetzt und aufgearbeitet. Der erhaltene ölige Rückstand ist auch durch mehrfaches Durchreiben mit kaltem Petrolether nicht festzubekommen und wurde daher nach Abziehen der Lösungsmittel direkt - wie in Beispiel 9 beschrieben -mit 5-Aminoallyl-dUTP zur Reaktion gebracht. Ausbeute: 0,25 g (Öl)

**Beispiel 9**

Digoxigenin-O-succinyl-γ-amidobutyryl- ε-amidocaproyl[5-amidoallyl)-2′-desoxy-uridin-5′-Triphosphate]-Tetralithiumsalz

(Dig-16-dUTP)

$C_{49}H_{70}O_{23}N_5P_3Li_4$ MG 1217,7

250 mg Digoxigenin-O-succinyl-γamidobutyryl-ε-amidocapronsäure-N-hydroxy-succinimid-ester (Öl aus Beispiel 8, ca. 0,3 mMol) werden in 7 ml DMF gelöst und zu einer Lösung von 210 mg 5-Allylamino-2′-desoxy-uridin-5′-triphosphat-Tetralithiumsalz (0,38 mMol) in 6 ml $H_2O$ gegeben. Man gibt dem Reaktionsgemisch 62 ml 0,2 m Natriumboratpuffer, pH 8,5 zu und rührt ca. 15 Stunden über Nacht bei Raumtemperatur. Der Reaktionsablauf wird wie unter Beispiel 4 beschrieben, verfolgt.

Zur Aufreinigung wird der Ansatz im Ölpumpenvakuum bis zum festen Rückstand eingedampft, in ca. 200 ml $H_2O$ gelöst und auf eine Ionenaustauschersäule (DEAE-Sephadex A-25, $Cl^-$-Form, Säulenabmessung 1,5 x 30 cm) gegeben. Nach Waschen mit $H_2O$ wird mit einem linearen Gradienten von 2 l $H_2O$ auf 2 l 0,3 m-LiCl eluiert. Die das reine Produkt enthaltenden Fraktionen werden vereinigt, im Vakuum so weit konzentriert, bis kein $H_2O$ mehr übergeht und das Konzentrat anschließend in einem Aceton-Ethanol-Gemisch (3:1) durch Einrühren gefällt. Man zentrifugiert vom Überstand ab, wäscht bis zur $Cl^-$-Freiheit mit Ethanol und trocknet mit Vakuum über $P_2O_5$/KOH. Ausbeute: 250 mg = 68 % der Th.

Analytik: $H_2O$-Bestimmung: 6,3%.

Elementaranalyse (unter Berücksichtigung des $H_2O$-Gehaltes):

| | | | |
|---|---|---|---|
| C ber.: 45,5% | H ber.: 5,7% | N ber.: 5,4% | P ber.: 7,2% |
| C gef.: 45,1% | H gef.: 5,6% | N gef.: 5,6% | P gef.: 7,0% |

UV-Spektrum (Phosphatpuffer pH 7,0):

Maxima:

220 nm (Schulter)

289 nm

**Beispiel 10**

Digoxigenin-O-succinyl-ε-amidocaproyl-[5-(amidoallyl)-uridin-5′-triphosphat]-Tetralithiumsalz

(Dig-11-UTP)

$C_{45}H_{63}O_{23}N_4P_3Li_4$ MG: 1148,5

Die Verbindung wird durch Umsetzung von 520 mg Digoxigenin-O-succinyl-ε-amidocapronsäure-N-hydroxysuccinimidester (0,74 mMol) mit 416,5 mg 5-Allylamino-UTP-Tetralithiumsalz (0,74 mMol) analog Beispiel 4 hergestellt. Die Ionenaustauscherchromatographie erfolgt jedoch in Abwandlung nach Beispiel 9 an DEAE-Sephadex-A-25 in der $Cl^-$-Form. Ausbeute: 560 mg = 66 % d. Th.

Analytik: $H_2O$-Bestimmung: 8,1%

Elementaranalyse (unter Berücksichtigung des $H_2O$-Gehaltes):

| | | | |
|---|---|---|---|
| C ber.: 43,5% | H ber.: 5,47% | N ber.: 4,5% | P ber.: 7,47% |
| C gef.: 43,1% | H gef.: 5,3% | N gef.: 4,5% | P gef.: 7,35% |

UV-Spektrum (Phosphatpuffer pH 7,0):

entspricht Dig-11-dUTP

**Beispiel 11**

Digoxigenin-O-succinyl-γ-amidobutyryl-ε-amidocaproyl-[5-(amidoallyl)-uridin-5′-triphosphat]-Tetralithiumsalz

(Dig-16-dUTP)

$C_{49}H_{70}O_{24}N_5P_3Li_4$ MG: 1233,7

Die Verbindung wird durch Umsetzung von 250 mg Digoxigenin-O-succinyl-γ-amidobutyryl- - amidocapronsäureN-hydroxysuccinimidester (0,3 mMol, nach Beispiel 8 erhalten) mit 214 mg 5-Allylamino-

UTP-$Li_4$ (0,38 mMol) analog Beispiel 9 hergestellt. Ausbeute: 218 mg = 59 % d. Th.
Analytik: $H_2O$-Bestimmung = 7,2%
Elementaranalyse (unter Berücksichtigung des $H_2O$-Wertes):

| | | | |
|---|---|---|---|
| C ber.: 44,45% | H ber.: 5,67% | N ber.: 5,3% | P ber.: 7,0% |
| C gef.: 44,3 % | H gef.: 5,5 % | N ber.: 5,3% | P gef.: 7,1% |

UV-Spektrum (Phosphatpuffer pH 7,0):
entspricht Dig-16-dUTP

**Beispiel 12**

Herstellung von N-Azidobenzoyl-1,8-diamino-3,6-dioxaoctan

5,20 g (20 mmol) Azidobenzoesäure-N-hydroxysuccinimidester (Fa. Pierce, D-6054 Rodgau 1) werden in wasserfreiem Essigester gelöst und mit 29,3 ml (200 mmol) 1,8-Diamino-3,6-dioxaoctan versetzt. Die Reaktionsmischung läßt man 20 Stunden bei 20° C im Dunkeln rühren. Das Lösungsmittel wird im Vakuum entfernt und der ölige Rückstand in 300 ml Wasser gelöst. Das Produkt wird mit 2 1 Toluol in einem Perforator aus der wäßrigen Phase extrahiert, wobei man die Apparatur mit Aluminiumfolie umwickelt. Die Extraktion ist nach ca. 16 Stunden beendet. Die organische Phase wird am Vakuum vom Lösungsmittel befreit und das Produkt durch präparative Säulenchromatographie an Kieselgel (Säule 80 x 10 cm, Eluent: Chloroform/Methanol/konz. Ammoniaklösung 65:30:5) aufgereinigt und nach Abdampfen des Lösungsmittels im Hochvakuum getrocknet. Ausbeute: 3,2 g (55%); farbloses, zähes Öl.

**Beispiel 13**

Herstellung von Digoxigenin-3-hemisuccinat[N′-(4-azidobenzoyl)]-8-amino-3,6-dioxaoctylamid (Photodigoxigenin)

2,93 g (10 mmol) des Produktes aus Beispiel 12 werden in 200 ml wasserfreiem Dioxan gelöst und mit 5,87 g (10 mmol) Digoxigenin-3-hemisuccinat-N-hydroxysuccinimidester (Herstellung analog: G. C. Oliver, Jr. Brent, M. Parker, D.L. Brasfield und Ch.W. Parker; J. clin. Invest. 47 (1986), 1035) versetzt. Man läßt 20 Stunden bei Raumtemperatur rühren, entfernt das Lösungsmittel im Vakuum und trennt das entstandene Produkt durch präparative Mitteldruck-Flüssig-Chromatographie ab (Säulenvolumen: 1640 ml, Labochrom Reversed-Phase-Silica HD-SIL-18-30-60, Eluent Methanol/Wasser 7:3 + 1 % Eisessig). Nach Sammlung der entsprechenden Fraktionen wird das Lösungsmittel im Vakuum entfernt und der ölige Rückstand in Dioxan Gelöst. Nach Lyophilisation und Waschen mit 100 ml Diisopropylether wird das Produkt Digoxigenin-3-hemisuccinat[N′-(4-azidobenzoyl)]8-amino-3,6-dioxaoctylamid als farbloser, leicht klebriger Feststoff erhalten, der im Hochvakuum getrocknet wird. Ausbeute: 4,9 g (64%).
IR (Aceton): = 2150, 1728, 1639 $cm^{-1}$.

**Beispiel 14**

Markierung einer Nukleinsäuresonde durch in vitro Transkription von RNA ("Transkriptions" Methode)

Das Prinzip der Reaktion ist der Fig. 4 zu entnehmen. Die zur Markierung verwendete DNA wurde in den Transkriptionsvektor pSPT18 (Fig. 5 und 7) insertiert. Der Vektor enthält einen Promotor für die SP6 und einen Promotor für die T7 RNA Polymerase. Die DNA wurde vor der Markierungsreaktion an einer Stelle außerhalb der insertierten DNA-Sequenz und der Promotoren, sowie den die Promotoren und die DNA-Sequenz verbindenden Sequenzen linearisiert.

1 µg der linearisierten Substrat-DNA wurde in einem Reaktionsgefäß je 1 µl einer 10 mmol/1 Lösung von Adenosintriphosphat, Cytidintriphosphat und Guanosintriphosphat zugesetzt. Dazu wurde 1 µl einer Lösung, die 6,5 mmol/1 Uridintriphosphat und 3,5 mmol/1 Digoxigenin-11-UTP enthält, zugegeben. Digoxigenin-11-UTP wird im Beispiel 10, ähnlich wie im Beispiel 4 Digoxigenin-11-dUTP hergestellt, als Ausgangssubstanz wird lediglich anstelle des Allylamino-Desoxyuridinsalzes das Allylamino-Uridinsalz eingesetzt.

Weiterhin wurden dem Ansatz 2 µl eines 10fach konzentrierten Puffers (Tris-HCl, 0,4 mol/1; $MgCl_2$, 60 mmol/1; Dithiothreitol, 50 mmol/1; Spermidin, 40 mmol/1; pH 7,2) zugegeben, das Volumen auf 19 µl mit sterilem bidestilliertem Wasser aufgefüllt und schließlich die Reaktion durch Zugabe von 1 µl, entsprechend 10 Einheiten der RNA Polymerase (SP6 bzw. T7) gestartet.

Nach kurzer Zentrifugation wurde der Ansatz bei 37° C für eine Stunde inkubiert.

Die Substrat-DNA wurde anschließend durch Zusatz von 1 µl DNASel, RNAse-frei, entsprechend 10 Einheiten, 15 Minuten bei 37° C abgebaut.

Die Reaktion wurde durch Zugabe von 2 µl 0,2 mol/1 EDTA, pH 8,0 gestoppt. Die erhaltene Digoxigenin-markierte RNA-Probe wurde durch Extraktion mit 1 Volumen Phenol und durch anschließende Ethanol-Präzipitation von Proteinen und Nukleotiden gereinigt und schließlich in sterilem Puffer (Tris-HCl, 10 mmol/1; EDTA, 1 mmol/1; pH 8,0) gelöst.

**Beispiel 15**

Sandwich-Hybridisierung mit Digoxigenin- und Biotinmarkierten Oligonukleotiden

a) Verwendete Oligonukleotide

Nachzuweisende Nukleinsäure: 95 mer

```
5'GGAGTGAGCG TTACGGTAGT TGTTCTTCCA AGCAACGGTC
  CAACCCAGAG CAGTACCGGA ACCGTCGGTA GCTGGCGCGC
  TGTCGTATCT ACCGG
```

Einfangsonde:
95 mer, 48 Basen komplementär zur nachzuweisenden Nukleinsäure

```
5'-CTGGG TTGGACCGTT GCTTGGAAGA ACAACTACCG
   TAACGCTCAC TCCGCTACCA CCTGGTCCGG TCAGTACGTT
   GGTGGCGCCG AAGCTCGTAT
```

Detektorsonde:
95 mer, 47 Basen komplementär zur nachzuweisenden Nukleinsäure.

```
5'-GTACTTACGA ATCCGCTGTT GGTAACGCTG AATCCCGTTA
   CGTTTTGACC GGTAGATACG ACAGCGCGCC AGCTACCGAC
   GGTTCCGGTA CTGCT
```

b) Photomarkierung der Oligonukleotide

Zu 10 µg Einfangsonde in 20 µl dest. Wasser wurden 20 µl 1 mg/ml Photobiotin (Forster et al. Nucl. Acids. Res. 13 (1985) 745-761) bei gedämpftem Licht gegeben. Das Gemisch wurde in einem offenen Eppendorf Reaktionsgefäß unter Eiskühlung mit einer Phillips HPLR 400 W Entladungslampe im Abstand 10 cm 20 Minuten lang bestrahlt.

Nach der Reaktion wurde mit 0,1 M Tris-HCl pH 9,0, 1,0 mM EDTA auf 100 µl aufgefüllt und zweimal mit Butan-2-ol extrahiert. Nach der zweiten Extraktion betrug das Volumen der wäßrigen Phase 30 bis 40 µl.

Nach Zugabe von Träger (Carrier) t RNA (ca. 1 µg) und 1/10 Volumen 3 M Ammoniumacetat wurde mit 3 Volumina Ethanol über Nacht bei -20 °C gefällt.

Das nach Abzentrifugieren bei 4 °C erhaltene Pellet wurde mit kaltem Ethanol gewaschen und getrocknet. Die markierte DNA wurde in 10 mM Tris-HCl, pH 7,5, 0,1 mM EDTA aufgenommen und bei 4 °C gelagert.

Die Markierung mit Digoxigenin erfolgte nach demselben Verfahren. Eine Lösung von 1 mg/ml Photodigoxigenin (Beispiel 13) in Dimethylformamid wurde eingesetzt. Zur Phasentrennung bei der Extraktion mit Butan-2-ol wurden 10 µl 5 M NaCl zugegeben.

c) Beschichten einer Membran mit Streptavidin

Verwendet wurde die Membran Nytran 13 N von Schleicher & Schüll.

In das Schleicher & Schüll Slot-Multifiltrationsgerät passende Membranstreifen wurden zugeschnitten und mit destilliertem Wasser angefeuchtet. Anschließend wurde die Membran in 1 mg/ml ThermoRSA (hergestellt nach EP 87 117 411.6) - Streptavidin für 30 Minuten bei Raumtemperatur mit Schütteln inkubiert. Die Membran wurde zweimal mit 0,1 M Tris-HCl, pH 7,5, 150 mM NaCl (Puffer 1) gewaschen.

Zur Absättigung der DNA-Bindungsstellen wurde 30 Minuten lang wie oben mit 3 mg/ml frisch denaturierter Heringsperm DNA inkubiert. Anschließend wurde zweimal mit Puffer 1 gewaschen und die Membran sofort in das Multifiltrationsgerät eingelegt.

d) Hybridisierung

| Reaktionsansatz: | Reaktionsvolumen 25 µl |
|---|---|
| Einfangsonde - (Biotin-markiert) | 5 ng |
| Detektorsonde - (Digoxigenin-markiert) | 5 ng |
| Puffer 1 | 5 x SSC = 0,5 M NaCl, 50 mM Na-citrat |
| nachzuweisende DNA | 2 pg - 1.000 ng |

Das Reaktionsgemisch wurde 5 Minuten bei 95 °C denaturiert, anschließend eine Stunde bei 68 °C inkubiert. Nach Abkühlen wurde das Reaktionsgemisch im Multifiltrationsgerät 15 Minuten auf der vorbehandelten Membran bei Raumtemperatur inkubiert. Danach war die Lösung in die Membran eingezogen. Es wurde kurz abgesaugt und dann zweimal mit je 50 µl Puffer 1 gewaschen.

e) Detektion des Sandwich DNA Komplexes

e1) Blockieren

Die Membran wurde aus dem Filtrationsgerät entnommen und 40 Minuten in 3% fettfreie Trockenmilch in Puffer 1 bei Raumtemperatur mit Schütteln inkubiert.

e2) Immunologische Reaktion

Die blockierte Membran wurde 30 Minuten in einer 1:3000 Verdünnung von Anti-Digoxigenin-Antikörper-Alkal. Phosphatase Konjugat in Puffer 1 bei Raumtemperatur mit Schütteln inkubiert.

e3) Waschen

Die Membran wurde dreimal mit Puffer 1 und einmal mit 0,1 M Tris-HCl, pH 9,5, 0,1 M NaCl, 50 mM $MgCl_2$ gewaschen.

e4) Farbreaktion

Die Membran wurde in einer frischen, mit Stickstoff gespülten Lösung von 0,33 mg/ml Nitro-Tetrazol-Blau und 0,167 mg/ml 5-Brom-4-Chlor-3-indolyl-phosphat unter Luftausschluß im Dunkeln bei Raumtemperatur inkubiert, am Besten in Folienbeutel eingeschweißt.

f) Ergebnisse

Mit diesem Verfahren konnten 10 bis 100 pg nachzuweisender Oligonukleotid-DNA in 25 µl Reaktionslösung nachgewiesen werden, das entspricht einer Konzentration von 4 ng/ml. Die Testdauer betrug ca. 6 Stunden. Die Auswertung einer Messung der Farbbildungsreaktion ist in Fig. 6 wiedergegeben.

Zugabe von bis zu 5000 mg/ml = 5 µg/ml Fremd DNA (Heringsperm) führt nicht zu einer Signalminderung.

Kontrolle Reaktionen:

Werden Sandwich Hybridisierungsansätze analog den beschriebenen auf eine Membran filtriert, die mit Thermo-RSA beschichtet wurde, wird ein background von 80 mE gemessen. Eine Abhängigkeit von der Menge sample - DNA wird nicht beobachtet.

Kontrollreaktionen, in denen entweder Biotin-Einfangsonden DNA oder Digoxigenin-Nachweissonden-DNA fehlen, zeigen ebenfalls background Werte von 80 mE.

## Ansprüche

1. Verfahren zum Nachweis von Nukleinsäuren definierter Sequenz durch Hybridisierung mit zwei in gleicher Lösungsphase vorliegenden, zu unterschiedlichen Bereichen der nachzuweisenden Nukleinsäure komplementären, einzelsträngigen Nukleinsäuresonden, wobei die eine Nukleinsäuresonde als Detektorsonde dient und über eine chemische Verbindung mindestens ein Hapten als Markierung gebunden enthält, und die andere Nukleinsäuresonde als Einfangsonde dient und an eine feste Matrix gebunden wird, **dadurch gekennzeichnet,** daß man als Hapten ein Steroid verwendet, das an mindestens eine Position der Detektorsonde, die nicht an der Wasserstoffbrückenbildung mit der nachzuweisenden Nukleinsäure beteiligt ist, über eine Brücke von mindestens vier Atomen Länge kovalent gebunden ist, die in Lösung vorliegende, nachzuweisende Nukleinsäure mit der Detektorsonde und der Einfangsonde in beliebiger Reihenfolge inkubiert, wobei man die Bindung der Einfangsonde an die Matrix vor, während oder nach der Inkubation mit der nachzuweisenden Nukleinsäure bewirkt, die verbleibende Lösung von dem matrixgebundenen Komplex aus Einfangsonde, nachzuweisender Nukleinsäure und Detektorsonde abtrennt und den Komplex über einen seinerseits markierten Anti-Hapten-Antikörper nachweist.

2. Verfahren nach Anspruch 1,
**dadurch gekennzeichnet,** daß man als Steroid Digoxigenin oder Digoxin verwendet.

3. Verfahren nach Anspruch 1 oder 2,
**dadurch gekennzeichnet,** daß die Einfangsonde adsorptiv, koordinativ, kovalent oder über ein Bindungssystem an die feste Matrix gekoppelt wird.

4. Verfahren nach Anspruch 3,
**dadurch gekennzeichnet,** daß das Bindungssystem aus zwei spezifisch miteinander bindefähigen Komponenten besteht, wobei eine Komponente an die Einfangsonde und die andere Komponente an die feste Matrix gebunden ist.

5. Verfahren nach Anspruch 4, **dadurch gekennzeichnet,** daß eine Komponente des Bindungssystems über eine Brücke von mindestens 4 Atomen Länge an mindestens eine Position der Einfangsonde, die nicht an der Wasserstoffbrückenbildung mit der nachzuweisenden Nukleinsäure beteiligt ist, gebunden ist.

6. Verfahren nach Anspruch 4 oder 5,
**dadurch gekennzeichnet,** daß die beiden Komponenten des Bindungssystems Biotin und (Strept-)Avidin sind.

7. Verfahren nach Anspruch 4 oder 5,
**dadurch gekennzeichnet,** daß die beiden Komponenten des Bindungssystems Oligo- oder Poly d(C) und Oligo oder Poly d(G) sind.

8. Verfahren nach Anspruch 4 oder 5,
**dadurch gekennzeichnet,** daß die beiden Komponenten des Bindungssystems ein Hapten und ein Anti-Hapten-Antikörper sind, mit der Maßgabe, daß das Hapten ein von der Markierung der Detektorsonde verschiedenes Hapten ist und der Anti-Hapten-Antikörper nicht mit dem Markierungshapten der Detektorsonde kreuzreagiert.

9. Verfahren nach Anspruch 4, **dadurch gekennzeichnet,** daß das Bindungssystem Oligo- oder Poly d(C)/Oligo- oder Poly d(G) ist und eine der beiden Komponenten sich direkt am 5′- oder 3′-Ende der Einfangsonde befinden.

10. Verfahren nach einem der Ansprüche 1 bis 9,
**dadurch gekennzeichnet,** daß eine Hapten-markierte Sonde verwendet wird, deren Hapten in die Nukleinsäure-Sonde chemisch im Rahmen der Oligo-Desoxyribonukleotid-Synthese durch Einbau geschützter, mit substituierbaren Aminofunktionen modifizierter Nukleosidphosphoamidite und - nach Entfernung der Schutzgruppen -durch Umsetzung des modifizierten Oligo-Desoxyribonukleotids mit aktivierten Estern, Amiden oder Ethern der Haptene eingebaut wurde.

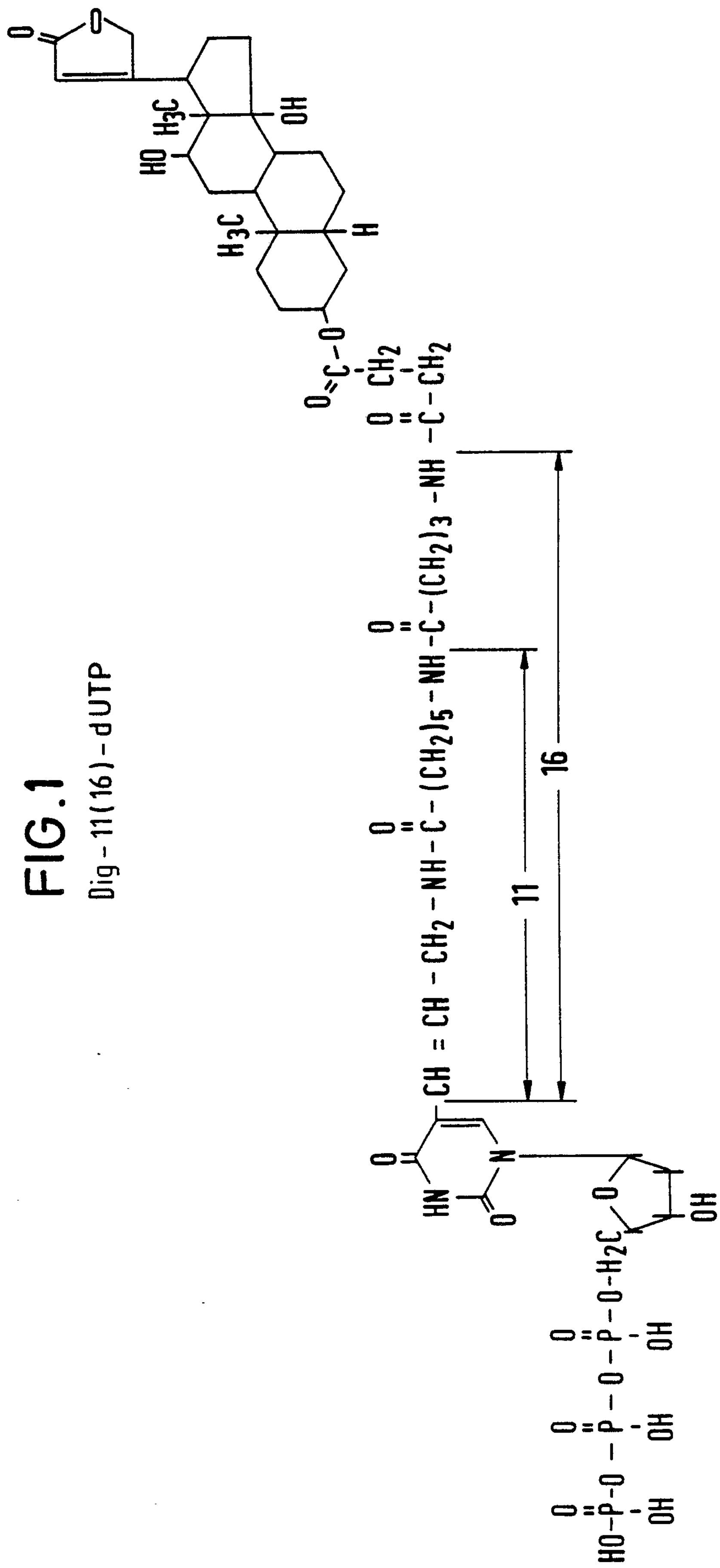

FIG.1

Dig-11(16)-dUTP

# FIG. 2

5 - Brom - 4 - chlor - 3 - indolylphosphat - $Na_2$

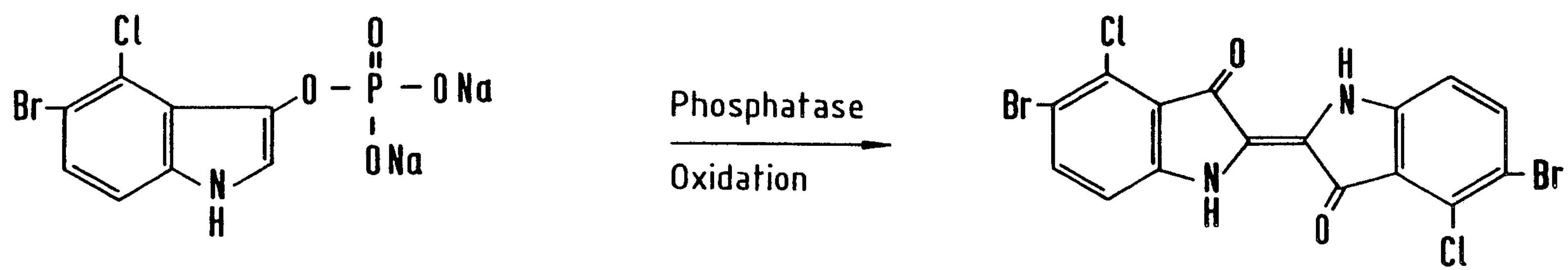


farblos

blau

ZU FIG. 2

Nitrotetrazolium Blue (NBT)
Syn.: 3,3'-(3,3'-Dimethoxy-4,4'-biphenylene)-bis-[5-phenyl-2-(4-nitrophenyl)-tetrazolium chloride]

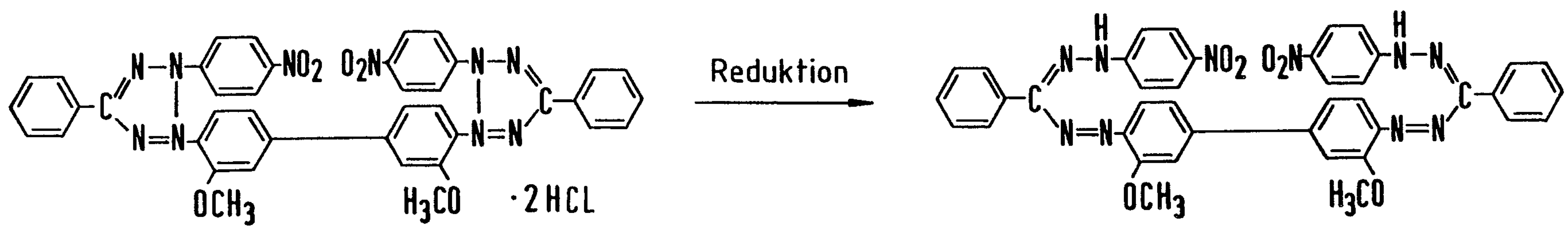


farblos

Tetrazoliumsalz

blau

Formazan

# FIG. 3

Digoxigenin - 3 - hemisuccinat - [N' - (4 - azidobenzoyl)] - 8 - amino - 3, 6 - dioxaoctylamid

Syntheseschema:

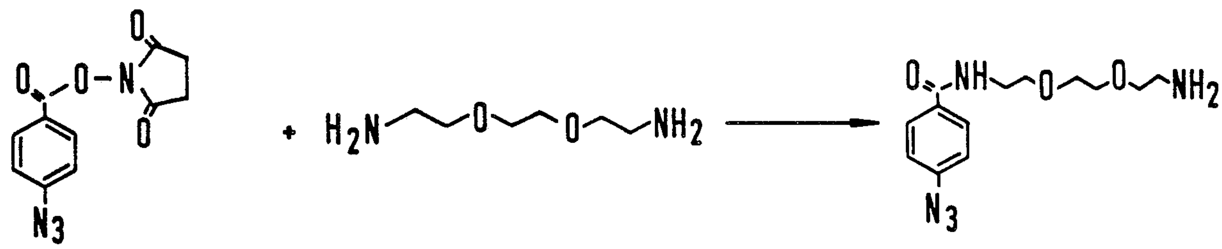

I II III

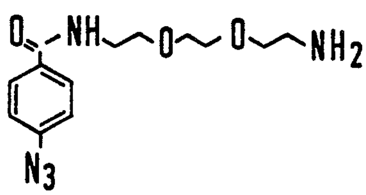

III

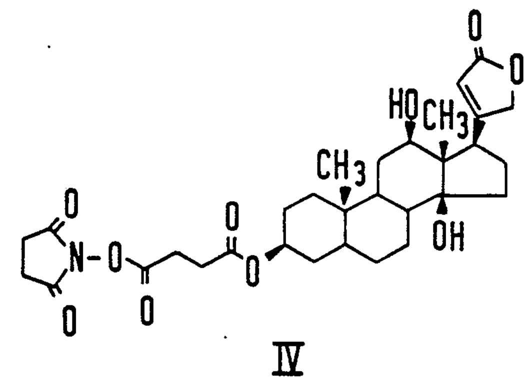

IV

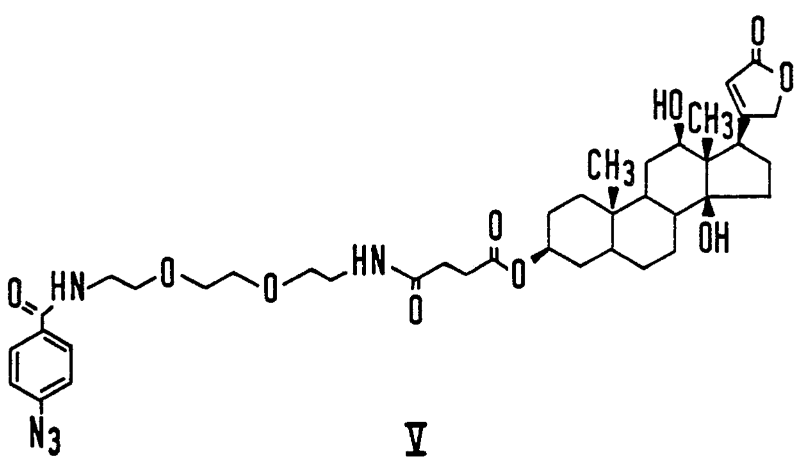

V

FIG.4

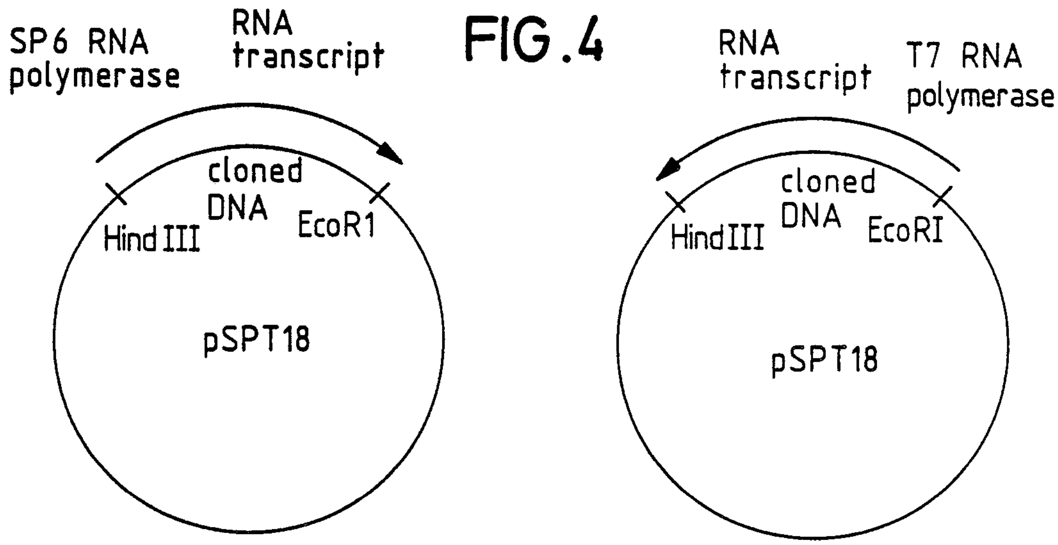
SP6 RNA polymerase
RNA transcript
cloned DNA
Hind III
EcoR1
pSPT18
RNA transcript
T7 RNA polymerase
cloned DNA
HindIII
EcoRI
pSPT18

FIG.5

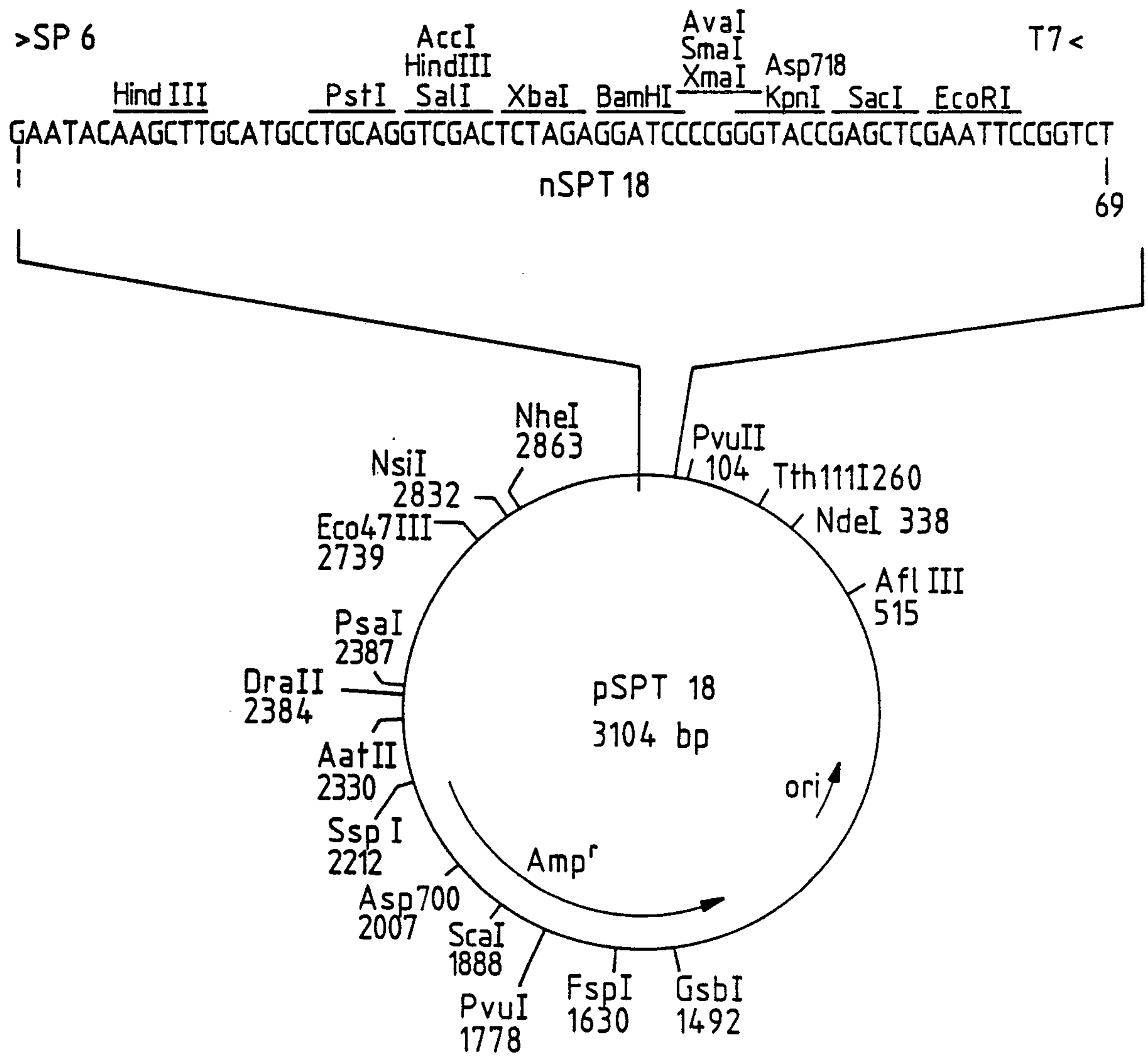
>SP 6
T7<
AccI
HindIII
SalI
AvaI
SmaI
XmaI
Asp718
KpnI
Hind III
PstI
XbaI
BamHI
SacI
EcoRI
GAATACAAGCTTGCATGCCTGCAGGTCGACTCTAGAGGATCCCCGGGTACCGAGCTCGAATTCCGGTCT
nSPT 18
69
NheI 2863
NsiI 2832
Eco47III 2739
PsaI 2387
DraII 2384
AatII 2330
Ssp I 2212
Asp700 2007
ScaI 1888
PvuI 1778
FspI 1630
GsbI 1492
PvuII 104
Tth111I260
NdeI 338
Afl III 515
pSPT 18
3104 bp
ori
Amp^r

FIG. 6

Farbbildungsreaktion bei der Bindung des Oligo-Sandwichs an Nitrozellulose-Streptavidin

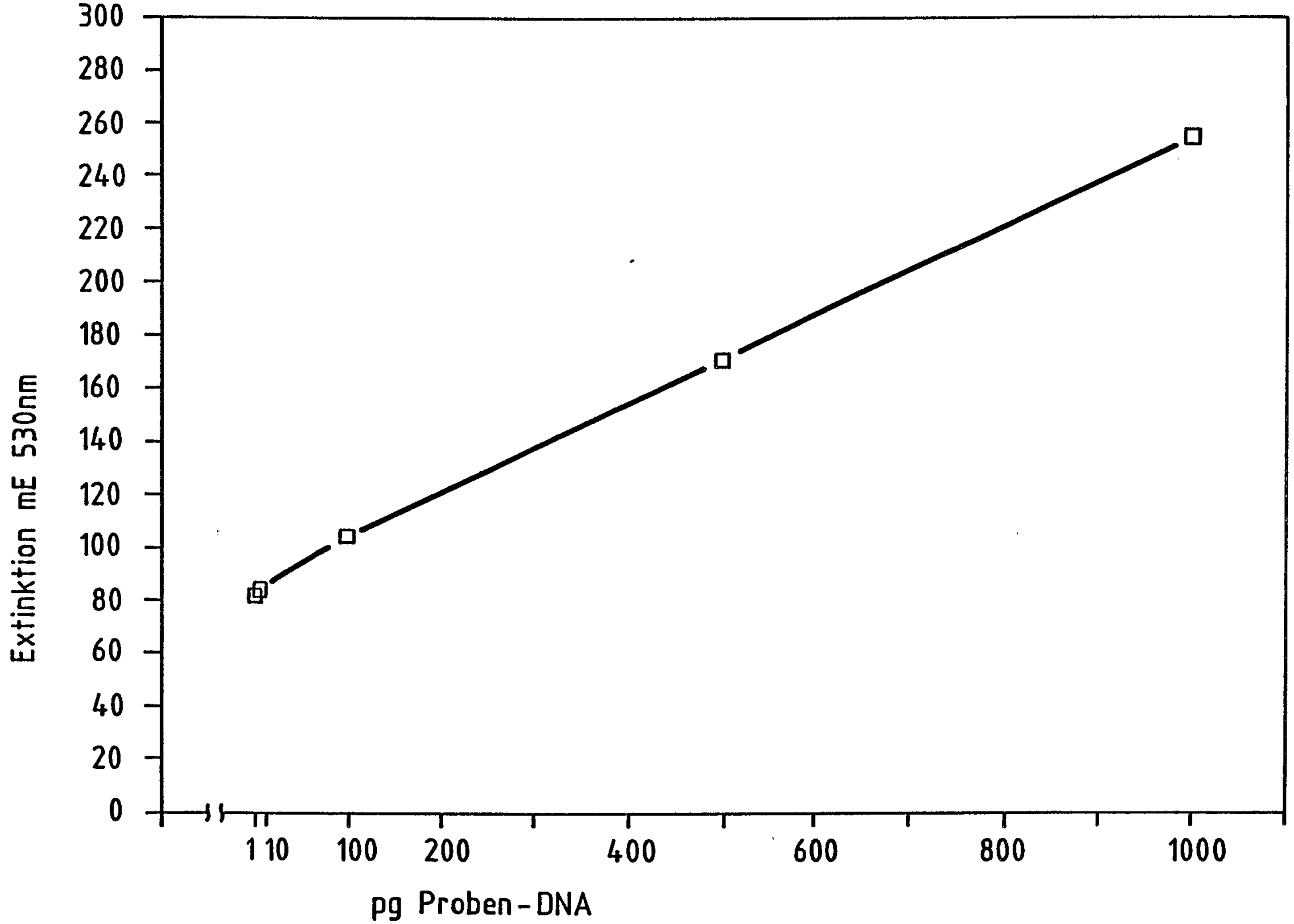

# FIG.7

DNA-Sequenz(A) und Restriktionskarte(B) von pSPT18

## FIG.7A

1 GAATACAAGC TTGCATGCCT GCAGGTCGAC TCTAGAGGAT CCCCGGGTAC

51 CGAGCTCGAA TTCCGGTCTC CCTATAGTGA GTCGTATTAA TTTCGATAAG

101 CCAGCTGGGC CTCGCGCGTT TCGGTGATGA CGGTGAAAAC CTCTGACACA

151 TGCAGCTCCC GGAGACGGTC ACAGCTTGTC TGTAAGCGGA TGCCGGGAGC

201 AGACAAGCCC GTCAGGGCGC GTCAGCGGGT GTTGGCGGGT GTCGGGGCGC

251 AGCCATGACC CAGTCACGTA GCGATAGCGG AGTGTATATA CTGGCTTAAC

301 TATGCGGCAT CAGAGCAGAT TGTACTGAGA GTGCACCATA TGCGGTGTGA

351 AATACCGCAC AGATGCGTAA GGAGAAAATA CCGCATCAGG CGCTCTTCCG

401 CTTCCTCGCT CACTGACTCG CTGCGCTCGG TCGTTCGGCT GCGGCGAGCG

451 GTATCAGCTC ACTCAAAGGC GGTAATACGG TTATCCACAG AATCAGGGGA

501 TAACGCAGGA AAGAACATGT GAGCAAAAGG CCAGCAAAAG GCCAGGAACC

551 GTAAAAAGGC CGCGTTGCTG GCGTTTTTCC ATAGGCTCCG CCCCCCTGAC

601 GAGCATCACA AAAATCGACG CTCAAGTCAG AGGTGGCGAA ACCCGACAGG

651 ACTATAAAGA TACCAGGCGT TTCCCCCTGG AAGCTCCCTC GTGCGCTCTC

701 CTGTTCCGAC CCTGCCGCTT ACCGGATACC TGTCCGCCTT TCTCCCTTCG

751 GGAAGCGTGG CGCTTTCTCA ATGCTCACGC TGTAGGTATC TCAGTTCGGT

801 GTAGGTCGTT CGCTCCAAGC TGGGCTGTGT GCACGAACCC CCCGTTCAGC

851 CCGACCGCTG CGCCTTATCC GGTAACTATC GTCTTGAGTC CAACCCGGTA

901 AGACACGACT TATCGCCACT GGCAGCAGCC ACTGGTAACA GGATTAGCAG

951 AGCGAGGTAT GTAGGCGGTG CTACAGAGTT CTTGAAGTGG TGGCCTAACT

1001 ACGGCTACAC TAGAAGGACA GTATTTGGTA TCTGCGCTCT GCTGAAGCCA

1051 GTTACCTTCG GAAAAAGAGT TGGTAGCTCT TGATCCGGCA AACAAACCAC

1101 CGCTGGTAGC GGTGGTTTTT TTGTTTGCAA GCAGCAGATT ACGCGCAGAA

1151 AAAAAGGATC TCAAGAAGAT CCTTTGATCT TTTCTACGGG GTCTGACGCT

1201 CAGTGGAACG AAAACTCACG TTAAGGGATT TTGGTCATGA GATTATCAAA

Fortsetzung FIG.7A

1251 AAGGATCTTC ACCTAGATCC TTTTAAATTA AAAATGAAGT TTTAAATCAA

1301 TCTAAAGTAT ATATGAGTAA ACTTGGTCTG ACAGTTACCA ATGCTTAATC

1351 AGTGAGGCAC CTATCTCAGC GATCTGTCTA TTTCGTTCAT CCATAGTTGC

1401 CTGACTCCCC GTCGTGTAGA TAACTACGAT ACGGGAGGGC TTACCATCTG

1451 GCCCCAGTGC TGCAATGATA CCGCGAGACC CACGCTCACC GGCTCCAGAT

1501 TTATCAGCAA TAAACCAGCC AGCCGGAAGG GCCGAGCGCA GAAGTGGTCC

1551 TGCAACTTTA TCCGCCTCCA TCCAGTCTAT TAATTGTTGC CGGGAAGCTA

1601 GAGTAAGTAG TTCGCCAGTT AATAGTTTGC GCAACGTTGT TGCCATTGCT

1651 ACAGGCATCG TGGTGTCACG CTCGTCGTTT GGTATGGCTT CATTCAGCTC

1701 CGGTTCCCAA CGATCAAGGC GAGTTACATG ATCCCCCATG TTGTGCAAAA

1751 AAGCGGTTAG CTCCTTCGGT CCTCCGATCG TTGTCAGAAG TAAGTTGGCC

1801 GCAGTGTTAT CACTCATGGT TATGGCAGCA CTGCATAATT CTCTTACTGT

1851 CATGCCATCC GTAAGATGCT TTTCTGTGAC TGGTGAGTAC TCAACCAAGT

1901 CATTCTGAGA ATAGTGTATG CGGCGACCGA GTTGCTCTTG CCCGGCGTCA

1951 ATACGGGATA ATACCGCGCC ACATAGCAGA ACTTTAAAAG TGCTCATCAT

2001 TGGAAAACGT TCTTCGGGGC GAAAACTCTC AAGGATCTTA CCGCTGTTGA

2051 GATCCAGTTC GATGTAACCC ACTCGTGCAC CCAACTGATC TTCAGCATCT

2101 TTTACTTTCA CCAGCGTTTC TGGGTGAGCA AAAACAGGAA GGCAAAATGC

2151 CGCAAAAAAG GGAATAAGGG CGACACGGAA ATGTTGAATA CTCATACTCT

2201 TCCTTTTTCA ATATTATTGA AGCATTTATC AGGGTTATTG TCTCATGAGC

2251 GGATACATAT TTGAATGTAT TTAGAAAAAT AAACAAATAG GGGTTCCGCG

2301 CACATTTCCC CGAAAAGTGC CACCTGACGT CTAAGAAACC ATTATTATCA

2351 TGACATTAAC CTATAAAAAT AGGCGTATCA CGAGGCCCTT TCGTCTCGCG

2401 CGTTTCGGTG ATGACGGTGA AAACCTCTGA CACATGCAGC TCCCGGAGAC

2451 GGTCACAGCT TGTCTGTAAG CGGATGCCGG GAGCAGACAA GCCCGTCAGG

2501 GCGCGTCAGC GGGTGTTGGC GGGTGTCGGG GCTGGCTTAA CTATGCGGCA

2551 TCAGAGCAGA TTGTACTGAG AGTGCACCAT ATCGACGCTC TCCCTTATGC

2601 GACTCCTGCA TTAGGAAGCA GCCCAGTAGT AGGTTGAGGC CGTTGAGCAC

2651 CGCCGCCGCA AGGAATGGTG CATGCAAGGA GATGGCGCCC AACAGTCCCC

2701 CGGCCACGGG CCTGCCACCA TACCCACGCC GAAACAAGCG CTCATGAGCC

2751 CGAAGTGGCG AGCCCGATCT TCCCCATCGG TGATGTCGGC GATATAGGCG

2801 CCAGCAACCG CACCTGTGGC GCCGGTGATG CCGGCCACGA TGCGTCCGGC

Fortsetzung **FIG .7A**

```
2851  GTAGAGGATC TGGCTAGCGA TGACCCTGCT GATTGGTTCG CTGACCATTT
2901  CCGGGTGCGG GACGGCGTTA CCAGAAACTC AGAAGGTTCG TCCAACCAAA
2951  CCGACTCTGA CGGCAGTTTA CGAGAGAGAT GATAGGGTCT GCTTCAGTAA
3001  GCCAGATGCT ACACAATTAG GCTTGTACAT ATTGTCGTTA GAACGCGGCT
3051  ACAATTAATA CATAACCTTA TGTATCATAC ACATACGATT TAGGTGACAC
3101  TATA
```

# FIG .7B

pSPT18 Spaltstellen

| Enzym | Zahl der Spaltstellen | Position der Spaltstellen | | | | |
|---|---|---|---|---|---|---|
| *Ava* II | 2 | 1546 | 1768 | | | |
| *Bgl* I | 2 | 106 | 1528 | | | |
| *Eco* 31I | 2 | 71 | 1469 | | | |
| *Fin* I | 2 | 2699 | 2908 | | | |
| *Hgi* EII | 2 | 335 | 1096 | | | |
| *Sph* I | 2 | 17 | 2674 | | | |
| *Apy* I | 3 | 543 | 664 | 677 | | |
| *Ban* II | 3 | 56 | 2750 | 2764 | | |
| *Bbe* I | 3 | 2688 | 2801 | 2822 | | |
| *Cfr* 10I | 3 | 1488 | 2821 | 2830 | | |
| *Dra* I | 3 | 1274 | 1293 | 1985 | | |
| *Eae* I | 3 | 1796 | 2702 | 2832 | | |
| *Eco* 57I | 3 | 1063 | 2075 | 2977 | | |
| *Eco* RII | 3 | 541 | 662 | 675 | | |
| *Gdi* II | 3 | 1796 | 2701 | 2832 | | |
| *Hae* I | 3 | 528 | 539 | 991 | | |
| *Mme* I | 3 | 730 | 914 | 2966 | | |
| *Nar* I | 3 | 2685 | 2798 | 2819 | | |
| *Tth* 111II | 3 | 1105 | 1112 | 1144 | | |
| *Apa* LI | 4 | 331 | 829 | 2075 | 2572 | |
| *Bsp* HI | 4 | 1235 | 2243 | 2348 | 2742 | |
| *Aha* II | 5 | 1945 | 2327 | 2685 | 2798 | 2819 |
| *Ban* I | 5 | 46 | 1356 | 2684 | 2797 | 2818 |
| *Fok* I | 5 | 201 | 1374 | 1555 | 1842 | 2485 |
| *Mae* I | 5 | 32 | 1010 | 1263 | 1598 | 2864 |
| *Mae* II | 5 | 266 | 1218 | 1634 | 2007 | 2327 |
| *Nsp* I | 5 | 17 | 152 | 519 | 2436 | 2674 |
| *Rsa* I | 5 | 48 | 323 | 1888 | 2564 | 3026 |
| *Sec* I | 5 | 41 | 42 | 675 | 2698 | 2704 |

Enzyme,die nicht schneiden

| | | | | |
|---|---|---|---|---|
| *Afl* II | *Apa* I | *Asu* II | *Avr* II | *Bal* I |
| *Bbv* II | *Bcl* I | *Bgl* II | *Bsm* I | *Bsp* MII |
| *Bss* HII | *Bst* EII | *Bst* XI | *Cla* I | *Dra* III |
| *Eco* RV | *Eco* R124 | *Esp* I | *Hpa* I | *Mlu* I |
| *Nco* I | *Not* I | *Nru* I | *Nsi* I | *Pfl* MI |
| *Pma* CI | *Ppu* MI | *Rsr* II | *Sac* II | *Sau* I |
| *Sfi* I | *Sna* I | *Sna* BI | *Spe* I | *Spl* I |
| *Stu* I | *Sty* I | *Xho* I | *Xma* III | |